# EUROPEAN PATENT APPLICATION

(11) **EP 4 018 995 A1**
(43) Date of publication of application: **29.06.2022**
(21) Application number: 21216284.6
(22) Date of filing: 21.12.2021
(51) Int. Cl.: A61K 8/35, C07C 1/00, A61Q 1/14, C07C 67/08

(54) **POLYGLYCEROL ESTERS OF PELARGONIC ACID**

(30) Priority: 22.12.2020 IT 202000031919
(71) Applicant: Roelmi HPC S.r.l, 21040 Origgio (VA) (IT)
(72) Inventor: MALANCHIN, Rosella, 21040 Origgio (VA) (IT); CARLOMAGNO, Federica, 21040 Origgio (VA) (IT); ORMELLESE, Giulia, 21040 Origgio(VA) (IT)
(74) Representative: Bianchetti & Minoja SRL

(57) **Abstract**

The present invention relates to cosmetic compositions containing mixtures of polyglycerol esters of pelargonic acid as solubilizers or emulsifiers.

## Description

The present invention relates to mixtures of polyglycerol esters of pelargonic acid obtainable by esterification between pelargonic acid and mixtures of polyglycerols, and to cosmetic compositions containing said mixtures of esters.

### Background of the invention

Cosmetic formulations typically contain a large number of ingredients, many of which are immiscible with one other, and therefore require the presence of emulsifying and/or solubilising agents with a long-lasting effect.

The scientific literature contains many examples describing the solubilising and/or emulsifying properties of polyglycerol esters of long-chain acids (including in the cosmetic field) having an even number of carbon atoms. In particular, Matsumura *et al.* (1999) describe the solubilising properties of polyglyceryl-3 caprate, while both Zotova *et al.* (1982, 1983) and Ezhov *et al.* (1974) describe the emulsifying properties of polyglyceryl-4 caprylate.

### State of the art

Bababyan V. K. et al. (Journal of the American Oil Chemists Society, Springer, DE, vol. 48 January 1971, p. 307-309) disclose the preparation and properties of some polyglycerol esters of short and medium chain length fatty acids, including pelargonic acid. These ester compounds are useful in stabilizing agents, moisturizers and softeners.

Pruvost R. et al. (Chemsuschem, vol. 8, no. 12, 2015, p. 2133-2137) disclose the palladium-catalyzed hydroesterification reaction with polyols and olefins in a liquid/liquid biphasic system. Glycerol was among the polyols that were reacted with 1-octene to yield a mixture of esters.

### Description of the invention

It has now been discovered that mixtures of polyglycerol esters of pelargonic acid obtainable by a process of esterification between pelargonic acid and a mixture of polyglycerols of Formula I, wherein n is zero or an integer, have solubilising and emulsifying properties surprisingly superior to those of equivalent mixtures derived from capric and caprylic acids.

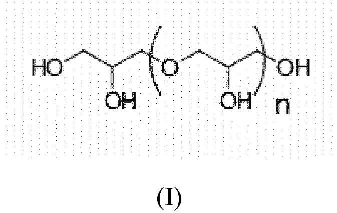

Accordingly, the invention provides a cosmetic composition containing a mixture of polyglycerol esters as a solubilizer or emulsifier, wherein said mixture is obtained by esterification between pelargonic acid and a mixture of polyglycerols of Formula I, wherein n ranges between 0 and 19, preferably between 0 and 6, said mixture of polyglycerols containing a fraction by weight of polyglycerol-4 or polyglycerol-6 which prevails over that of the other polyglycerols.

In one embodiment, the mixture of polyglycerols contains a fraction by weight of polyglycerol-6 which prevails over that of the other polyglycerols.

In a preferred embodiment, said mixture of polyglycerol contains a fraction by weight of polyglycerol-4 which is more abundant than that of other polyglycerol fractions.

In a particularly preferred embodiment, the mixture of polyglycerols contains at least 50% by weight of polyglycerol-4.

The word "mixture" as used herein should not be interpreted only as a "mixture" as defined in Regulation (EC) no. 1907/2006 and subsequent revisions, ie. as a mixture of various raw materials present in isolated form. As exemplified in Examples 1, 2 and 3, the polyglycerol mixtures used can be obtained directly from natural products containing polyglycerols having different glycerol units without said polyglycerols having been individually isolated before the formation of the mixture.

The cosmetic compositions according to the invention can take any form acceptable and suitable for a cosmetic composition. They can therefore take the form of a suspension; dispersion, and in particular vesicle-based oil-in-water dispersion; organic or oily solution optionally thickened or also gelled; oil-in-water, water-in-oil or multiple emulsion; gel or mousse; oily or emulsified gel; dispersion of vesicles, in particular lipid vesicles; two-phase or multi-phase lotion; spray; lotion; cream; ointment; fatty ointment.

In a further aspect, the invention provides the use of a mixture of polyglycerol esters as defined above in any embodiment, as a solubilizer or emulsifier for the preparation of a cosmetic composition.

The invention will be further illustrated by the following examples.

### EXAMPLES

### Example 1 (comparative example not part of the invention): Synthesis of a mixture of polyglycerol esters of pelargonic acid (PEL-PG3) starting with a mixture of polyglycerols having a prevalent fraction of polyglycerol-3

40 g of a mixture of polyglycerols having the specifications listed in Table 1, 13.175 g of pelargonic acid and 10 g of demineralised water are weighed in a 100 ml two-necked flask. The flask is placed under mechanical stirring in a nitrogen atmosphere and heated to a temperature ranging between 130°C and 150°C, distilling the water. The reaction is catalysed with 0.5% of paratoluenesulphonic acid (TsOH) and continued until an acid value number of 0.1 mg KOH/g is reached, corresponding to a time of about 3 hours. The water residue is then distilled under vacuum, and the product is left to cool at room temperature, maintaining the degree of vacuum.

The amount of the individual polyglycerol esters of pelargonic acid contained in said mixture can be evaluated with the HPLC-Mass analysis technique.

**Table 1**

| **Product name "Pure Vegetable Polyglycerine-3"** | | | **Product code SP-PG3** |
|---|---|---|---|
| **Compound** | **UNIT** | **Specifications** | |
| | **%** | **MIN** | **MAX** |
| **Glycerol** | **%** | | **≤1** |
| **Diglycerol** | **%** | **≥15** | **≤30** |
| **Triglycerol** | **%** | **≥35** | **≤48** |
| **Tetraglycerol** | **%** | **≥10** | **≤25** |
| **Pentaglycerol** | **%** | | **≤10** |
| **Heptaglycerol and higher** | **%** | | **≤10** |
| **Di+tri+tetraglycerol** | **%** | **≥*75*** | |
| **Water** | | | **≤ 0.5** |
| **Colour** | **Hazen** | | **≤ 50** |
| **Fatty acids and esters** | **mEq/100g** | | **≤ 0.8** |
| **Chlorides** | **ppm** | | **≤ 5** |
| **Sulphated ash** | **%** | | **≤ 0.05** |

### Example 2 - Synthesis of a mixture of polyglycerol esters of pelargonic acid (PEL-PG4) starting with a mixture of polyglycerols having a prevalent fraction of polyglycerol-4

40 g of a mixture of polyglycerols having the specifications listed in Table 2, 10 g of pelargonic acid and 10 g of demineralised water are weighed in a 100 ml two-necked flask. The flask is placed under mechanical stirring in a nitrogen atmosphere and heated to a temperature ranging between 130°C and 150°C, distilling the water. The reaction is catalysed with 0.5% of paratoluenesulphonic acid (TsOH) and continued until an acid value number of 0.1 mg KOH/g is reached, corresponding to a time of about 3 hours. The water residue is then distilled under vacuum, and the product is left to cool at room temperature, maintaining the vacuum level.

The amount of the individual polyglycerol esters of pelargonic acid contained in said mixture can be evaluated with the HPLC-Mass analysis technique.

**Table 2**

| **Product name "Pure Vegetable Polyglycerine-4"** | | | **Product code SP-PG4** |
|---|---|---|---|
| **Compound** | **UNIT** | **Specifications** | |
| | **%** | **MIN** | **MAX** |
| **Glycerol** | **%** | | **≤1** |
| **Diglycerol** | **%** | | **≤10** |
| **Tri+Tetraglycerol** | **%** | **≥65** | |
| **Di+Tri+Tetraglycerol** | **%** | **≥75** | |
| **Tetraglycerol and higher** | **%** | **≥50** | |
| **Heptaglycerol and higher** | **%** | | **≤10** |
| **Water** | | | **≤ 0.5** |
| **Colour** | **Hazen** | | **≤ 50** |
| **Fatty acids and esters** | **mEq/100g** | | **≤ 0.8** |
| **Chlorides** | **ppm** | | **≤ 5** |
| **Sulphated ash** | **%** | | **≤ 0.05** |

### Example 3 - Synthesis of a mixture of polyglycerol esters of pelargonic acid (PEL-PG6) starting with a mixture of polyglycerols having a prevalent fraction of polyglycerol-6

40 g of a mixture of polyglycerols having the specifications listed in **Table 3,** 6.85 g of pelargonic acid and 10 g of demineralised water are weighed in a 100 ml two-necked flask. The flask is placed under mechanical stirring in a nitrogen atmosphere and heated to a temperature ranging between 130°C and 150°C, distilling the water. The reaction is catalysed with 0.5% of paratoluenesulphonic acid (TsOH) and continued until an acid value number of 0.1 mg KOH/g is reached, corresponding to a time of about 3 hours. The water residue is then distilled under vacuum, and the product is left to cool at room temperature, maintaining the degree of vacuum.

The amount of the individual polyglycerol esters of pelargonic acid contained in said mixture can be evaluated with the HPLC-Mass analysis technique.

**Table 3**

| **Product name "Pure Vegetable Polyglycerine-6"** | | | **Product code SP-PG6** |
|---|---|---|---|
| **Compound** | **UNIT** | **Specifications** | |
| | **%** | **MIN** | **MAX** |
| **Glycerol** | **%** | | **≤1** |
| **Diglycerol** | **%** | | **≤10** |
| **Tetraglycerol and higher** | **%** | **≥50** | |
| **Hexaglycerol and higher** | **%** | **≥20** | |
| **Water** | | | **≤ 0.5** |
| **Colour** | **Hazen** | | **≤ 100** |
| **Fatty acids and esters** | **mEq/100g** | | **≤ 0.8** |
| **Chlorides** | **ppm** | | **≤ 5** |
| **Sulphated ash** | **%** | | **≤ 0.05** |

### Example 4 - Method for making polyglycerols

High molecular weight polyglycerols are prepared through an acid-catalyzed polymerization of an inexpensive, non-toxic, and renewable monomer: glycerol.

The polymerization protocol described herein can be performed at modest temperatures using standard polymerization equipment.

This method includes heating glycerol at a temperature of at least about 120 °C at a pressure below about 0.5 bar in the absence of a glyceride and the presence of a catalytic amount of acid selected from sulfuric acid, triflic acid, hydrochloric acid, hexafluorophosphoric acid, and tetrafluoroboric acid, or mixtures thereof.

The reaction scheme below shows an example of the preparation of polyglycerols starting from glycerin.

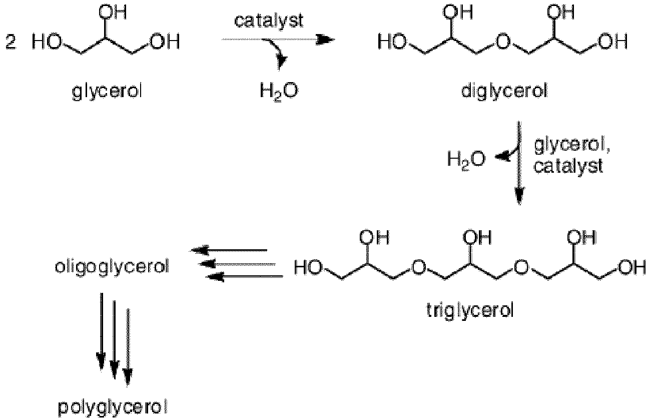

### Example 5 - Comparison tests with polyglycerol esters of capric and caproic acids.

The mixtures PEL-PG3, PEL-PG4 and PEL-PG6, obtained as described in Examples 1, 2 and 3 above, were examined with the analogous mixtures of capric acid (C10-PG3) and caprylic acid (C8-PG4), which were obtained by esterifying said acids with procedures similar to those of Examples 1-3, namely by using mixtures of polyglycerols having the same characteristics as those of Examples 1 and 2 respectively.

### A- Tests of solubilisation of essential oil of lemon 9:1

Procedure: Weigh 9 grams of polyglycerol ester mixture in a 150 ml beaker. Add 1 gram of essential oil of lemon to the beaker and stir slowly until homogenisation is complete. Slowly add 90 grams of osmosis water, maintaining moderate stirring. Continue stirring for 30 minutes until homogenisation is complete.

| **Ingredient** | **Concentration [w/w]** | **Result** |
|---|---|---|
| PEL-PG3 | 9 | The composition is opaque at time zero. After 24 hours it is viscous and with a lot of bubbles. |
| Essential oil of lemon | 1 | |
| Water | 90 | |

| **Ingredient** | **Concentration [w/w]** | **Result** |
|---|---|---|
| PEL-PG4 | 9 | The composition is transparent at time zero. 24 hours later, the composition is still clear. No separations or haloes are observed on the surface. |
| Essential oil of lemon | 1 | |
| Water | 90 | |

| **Ingredient** | **Concentration [w/w]** | **Result** |
|---|---|---|
| PEL-PG6 | 9 | The composition is transparent at time zero. 24 hours later, the composition is still clear. No separations or haloes are observed on the surface. |
| Essential oil of lemon | 1 | |
| Water | 90 | |

| **Ingredient** | **Concentration [w/w]** | **Result** |
|---|---|---|
| C10-PG3 | 9 | The composition is opaque at time zero. After 24 h, strong phase separation is observed, consisting of two transparent but clearly separate parts |
| Essential oil of lemon | 1 | |
| Water | 90 | |

| **Ingredient** | **Concentration [w/w]** | **Result** |
|---|---|---|
| C8-PG4 | 9 | The composition is opaque at time zero. After 24 h the composition is still opaque, with a slight deposit at the bottom. |
| Essential oil of lemon | 1 | |
| Water | 90 | |

### B- Tests of solubilisation of essential oil of lemon 5:1

Procedure: weigh 5 grams of polyglycerol ester mixture in a 150 ml beaker. Add 1 gram of essential oil of lemon to the beaker and stir slowly until homogenisation is complete. Slowly add 94 grams of osmosis water, maintaining moderate stirring. Continue stirring for 30 minutes until homogenisation is complete.

| **Ingredient** | **Concentration [w/w]** | **Result** |
|---|---|---|
| PEL-PG3 | 5 | The composition is semitransparent at time zero, with few bubbles. 24 hours later, the composition remains the same. |
| Essential oil of lemon | 1 | |
| Water | 94 | |

| **Ingredient** | **Concentration [w/w]** | **Result** |
|---|---|---|
| PEL-PG4 | 5 | The composition is transparent at time zero. 24 hours later, the composition is still clear. No separations or haloes are observed on the surface. |
| Essential oil of lemon | 1 | |
| Water | 94 | |

| **Ingredient** | **Concentration [w/w]** | **Result** |
|---|---|---|
| PEL-PG6 | 5 | The composition is transparent at time zero. 24 hours later, the composition is still clear. No separations or haloes are observed on the surface. |
| Essential oil of lemon | 1 | |
| Water | 94 | |

| **Ingredient** | **Concentration [w/w]** | **Result** |
|---|---|---|
| C10-PG3 | 5 | The composition is opaque at time zero. After 24 it is opaque, and no phase separation consisting of two transparent but clearly separate parts is observed |
| Essential oil of lemon | 1 | |
| Water | 94 | |

| **Ingredient** | **Concentration [w/w]** | **Result** |
|---|---|---|
| C8-PG4 | 5 | The composition is opaque at time zero. After 24 hours it is opaque |
| Essential oil of lemon | 1 | |
| Water | 94 | |

### C- Tests of solubilisation of essential oil of lemon 3:1

Procedure: weigh 3 grams of polyglycerol ester mixture in a 150 ml beaker. Add 1 gram of essential oil of lemon to the beaker and stir slowly until homogenisation is complete. Slowly add 96 grams of osmosis water, maintaining moderate stirring. Continue stirring for 30 minutes until homogenisation is complete.

| **Ingredient** | **Concentration [w/w]** | **Result** |
|---|---|---|
| PEL-PG3 | 3 | The composition is opaque at time zero. 24 hours later, the composition is still opaque. |
| Essential oil of lemon | 1 | |
| Water | 96 | |

| **Ingredient** | **Concentration [w/w]** | **Result** |
|---|---|---|
| PEL-PG4 | 3 | The composition is transparent at time zero. 24 hours later, the composition is still clear. No separations or haloes are observed on the surface. |
| Essential oil of lemon | 1 | |
| Water | 96 | |

| **Ingredient** | **Concentration [w/w]** | **Result** |
|---|---|---|
| PEL-PG6 | 3 | The composition is transparent at time zero. 24 hours later, the composition is still clear. No separations or haloes are observed on the surface. |
| Essential oil of lemon | 1 | |
| Water | 96 | |

| **Ingredient** | **Concentration [w/w]** | Result |
|---|---|---|
| C10-PG3 | 3 | The composition is opaque at time zero. After 24 hours separation into two phases, both opaque, is observed. |
| Essential oil of lemon | 1 | |
| Water | 96 | |

| **Ingredient** | **Concentration [w/w]** | **Result** |
|---|---|---|
| C8-PG4 | 3 | The composition is opaque at time zero. After 24 hours it is opaque. |
| Essential oil of lemon | 1 | |
| Water | 96 | |

### Example 6 - Cleansing tests

The detergent effect of aqueous solutions with different contents (3%, 5%, 7% and 10% by weight) of PEL-PG4 and PEL-PG6 of Examples 2 and 3 respectively, was tested.

Six make-up products were used to evaluate the make-up removal efficacy of the polyglycerol esters (mascara, pressed powders, baked powders, lipstick, liquid lipstick and liquid eyeshadow).

The tests were conducted by five operators, with a total of 10 repeats per operator.

### Method:

The six make-up products were applied to the arm, and left for 30 minutes to dry completely.

A make-up remover pad was soaked with make-up removal product, and wiped three times over the arm area marked by the make-up.

A score was assigned to each test according to the following key:
o (blank): make-up not removed
o X: make-up removed

| | **Aqueous solution containing 3% PEL-PG4** | **Aqueous solution containing 5% PEL-PG4** | **Aqueous solution containing 7% PEL-PG4** | **Aqueous solution containing 10% PEL-PG4** |
|---|---|---|---|---|
| **MASCARA** | **x** | **x** | **x** | **x** |
| **PRESSED POWDERS** | **x** | **x** | **x** | **x** |
| **BAKED POWDERS** | **x** | **x** | **x** | **x** |
| **LIPSTICK** | **x** | **x** | **x** | **x** |
| **LIQUID LIPSTICK** | **x** | **x** | **x** | **x** |
| **LIQUID EYESHADOW** | **x** | **x** | **x** | **V** |

| | **Aqueous solution containing 3% PEL-PG6** | **Aqueous solution containing 5% PEL-PG6** | **Aqueous solution containing 7% PEL-PG6** | **Aqueous solution containing 10% PEL-PG6** |
|---|---|---|---|---|
| **MASCARA** | | | **x** | **x** |
| **PRESSED POWDERS** | **x** | **x** | **x** | **x** |
| **BAKED POWDERS** | **x** | **x** | **x** | **x** |
| **LIPSTICK** | | **x** | **x** | **x** |
| **LIQUID LIPSTICK** | | | **x** | **x** |
| **LIQUID EYESHADOW** | | | **x** | **V** |

The results demonstrate that the tested products possess make-up removal properties. The best performing product was PEL-PG4.

### Example 7 - Detergent milk containing a mixture of Example 2

| | % |
|---|---|
| **Phase A** | |
| Water | 69.50 |
| Glycerin | 3.80 |
| Tetrasodium ethylenediaminetetraacetate (EDTA) | 0.05 |
| Water, benzyl alcohol, sodium benzoate, potassium sorbate | 0.80 |
| Glycerin, inulin lauroyl carbamate | 0.60 |
| PEL-PG4 | 6.00 |
| **Phase A1** | |
| Xanthan gum | 0.20 |
| **Phase B** | |
| Glyceryl stearate, cetylstearyl alcohol, stearic acid, sodium lauroyl glutamate | 3.00 |
| Cetyl alcohol | 2.50 |
| Prunus amygdalus dulcis oil | 6.10 |
| Triolein, glycerin dioleate | 6.00 |
| Phenoxy ethanol | 0.30 |
| Triglycerides C10-18 | 1.00 |
| **Phase C** | |
| Perfume | 0.15 |
| Total | 100.00 |

Procedure:
1) Prepare phase A by adding the ingredients in the order indicated, and heat to about 70°C
2) Prepare phase B by adding the ingredients in the order indicated, and heat to about 70°C
3) At 70°C slowly add phase A1 and stir until a homogeneous solution is obtained
4) Add phase B to phase A+A1 and stir with turbine.
5) Leave to cool under stirring and add phase C.

### Example 8 - Micellar water containing a mixture of Example 2

| | % |
|---|---|
| **Phase A** | |
| Water | 43.95 |
| Grapefruit extract | 40.00 |
| Glycerin | 3.00 5 |
| Aloe barbadensis leaf juice | 1.00 |
| Sodium hyaluronate | 0.25 |
| Pentylene glycol | 5.00 |
| Sodium benzoate | 0.30 |
| **Phase B** | |
| PEL-PG4 | 6.00 |
| Perfume | 0.50 |
| Total | 100.00 |

Procedure:
1) Prepare phase A, adding the ingredients in the indicated order at room temperature, under stirring
2) Prepare phase B under stirring, heating if necessary to obtain a homogeneous phase
3) Add phase B to phase A under stirring at room temperature
4) Stir until a clear solution is obtained

### Bibliography

Matsumura et al., Nihon Yukagakkaishi (1999), 48(7), 681-692, DOI 1 0.5650/jos 1996.48.681
Zotova et al, Silik. Mater. Miner. Syr'ya (1983), 86-92. Editor(s): Krasheninnikov, O. N. Publisher: Izd. Nauka, Leningr. Otd., Leningrad, USSR
Zotova et al., Kolloidnyi Zhurnal (1982), 44(1), 126-9
Ezhov *et al.,* Peny, Poluch. Primen., Mater. Vses. Nauchno-Tekh. Konf., 1st
(1974), Meeting Date 1973, Volume 1, 82-7 Publisher: Akad. Nauk SSSR, Inst. Fiz.Khim., Moscow, USSR

## Claims

1. A cosmetic composition containing a mixture of polyglycerol esters as a solubilizer or emulsifier, wherein said mixture is obtained by esterification between pelargonic acid and a mixture of polyglycerols of Formula (I), 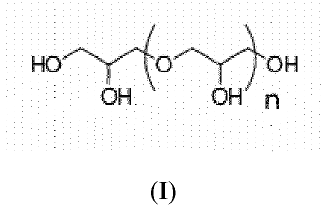 wherein n ranges between 0 and 19, preferably between 0 and 6, said mixture of polyglycerols containing a fraction by weight of polyglycerol-4 or polyglycerol-6 which prevails over that of the other polyglycerols.

2. A cosmetic composition according to claim 1, wherein said mixture of polyglycerols contains a polyglycerol-4 fraction by weight which is prevalent with respect to that of the other polyglycerols.

3. A cosmetic composition according to claim 2, wherein said mixture of polyglycerols contains at least 50% by weight of polyglycerol-4.

4. A cosmetic composition according to claim 1, wherein said mixture of polyglycerols contains a polyglycerol-6 fraction by weight which is prevalent with respect to that of the other polyglycerols.

5. A cosmetic composition according to claims 1-4 in the form of a lotion, cream or ointment.

6. The use of a mixture of polyglycerol esters as defined in claims 1-4, as a solubilizer or emulsifier in the preparation of a cosmetic composition.
